# EUROPEAN PATENT SPECIFICATION

(11) **EP 2 077 851 B1**
(45) Date of publication and mention of the grant of the patent: **20.10.2010**
(21) Application number: 07833029.7
(22) Date of filing: 21.09.2007
(51) Int. Cl.: A61K 31/7076, A61P 3/04

(54) **A PHARMACEUTICAL COMPOSITION COMPRISING CORDYCEPIN FOR THE TREATMENT AND PREVENTION OF OBESITY**
PHARMAZEUTISCHE ZUSAMMENSETZUNG enthaltend CORDYCEPIN ZUR BEHANDLUNG UND PRÄVENTION VON FETTleibigkeit
COMPOSITION PHARMACEUTIQUE COMPRENANT de LA CORDYCEPINE pour LE TRAITEMENT ET LA PRÉVENTION DE L'OBÉSITÉ

(30) Priority: 26.09.2006 KR 20060093732
(43) Date of publication of application: 15.07.2009
(73) Proprietor: Konkuk University Industrial Cooperation Corp., Seoul 137-753 (KR)
(72) Inventor: KIM, Si Kwan, Chungcheongbuk-do 380-180 (KR); KIM, Sung Won, Chungcheongbuk-do 380-754 (KR); LEE, Su Chan, Ulsan 680-803 (KR); KIM, Il Woung, Chungcheongbuk-do 380-150 (KR)
(74) Representative: Hart-Davis, Jason
(86) International application number: PCT/KR2007/004668
(87) International publication number: WO 2008/038973

(56) References cited:
- JP-A- 2001 131 196
- KR-A- 20010 054 264
- KR-A- 20030 039 981
- KR-A- 20050 063 164
- SHIMADA TSUYOSHI ET AL: "Suppression of adipocyte differentiation by Cordyceps militaris through activation of the aryl hydrocarbon receptor" AJP ENDOCRINOLOGY AND METABOLISM, vol. 295, October 2008 (2008-10), pages E859-E867, XP002553708
- KIM H G ET AL: "Cordycepin inhibits lipopolysaccharide-induced inflammation by the suppression of NF-kappaB through Akt and p38 inhibition in RAW 264.7 macrophage cells" EUROPEAN JOURNAL OF PHARMACOLOGY, ELSEVIER BV, NL, vol. 545, no. 2-3, 18 September 2006 (2006-09-18), pages 192-199, XP025169849 ISSN: 0014-2999 [retrieved on 2006-09-18]
- DATABASE WPI Week 200658 Thomson Scientific, London, GB; AN 2006-566031 XP002553712 & KR 2005 063 164 A (KIM K J) 28 June 2005 (2005-06-28)
- DATABASE WPI Week 20027 Thomson Scientific, London, GB; AN 2002-053633 XP002553713 & KR 2001 054 264 A (AHN Y J) 2 July 2001 (2001-07-02)
- DATABASE WPI Week 200377 Thomson Scientific, London, GB; AN 2003-826115 XP002553714 & KR 2003 039 981 A (INJE EDUCATIONAL FOUND) 22 May 2003 (2003-05-22)
- DATABASE WPI Week 200156 Thomson Scientific, London, GB; AN 2001-511255 XP002553715 & KR 2001 017 055 A (KIM J S) 5 March 2001 (2001-03-05)
- DATABASE WPI Week 200676 Thomson Scientific, London, GB; AN 2006-729283 XP002553709 & CN 1 765 381 A (TAN Y) 3 May 2006 (2006-05-03)
- SUGAR A.M. ET AL.: 'Antifungal Activity of 3'-Deoxyadenosine Cordycepin)' ANTIMICROBIAL AGENTS AND CHEMOTHERAPY vol. 42, no. 6, June 1998, pages 1424 - 1427, XP008109945

## Description

### Technical Field

The present invention relates to a pharmaceutical composition for the treatment and prevention of obesity, comprising cordycepin as an active ingredient. More specifically, the present invention relates to a pharmaceutical composition comprising cordycepin that suppresses the expression levels of and C/EBPα, PPARγ and anti-leptin, thereby inhibiting the differentiation of fibroblast cells (3T3-L1 cells) into adpocytes and the biosynthesis of triglyacyglycerol in the cell.

### Background Art

Obesity refers to a body condition in which natural energy reserves, stored in the fatty tissues of humans, are excessively increased in proportional to body weight. With more rapid, complex and varied changes in lifestyle than ever, modern people are prone to become obese due to bad lifestyle habits, imbalance of ingested nutrients, exercise deficiency, and excessive drinking.

Under the increasing influence of Western culture, Asians, who have been accustomed to vegetables and carbohydrate as their main diet, are changing in lifestyle with regard to diet, eating habit and residence, in the direction of increasing morbidity from obesity.

Obesity has been shown to cause various diseases, particularly hypertension, heart disease, diabetes mellitus, etc., and to increase the onset of chronic diseases, thus leading to a significant reduction in the lifespan of obese persons.

As is the case with many medical conditions, the caloric imbalance that results in obesity often develops from a combination of genetic and environmental factors. Particularly, obesity is greatly influenced by heredity. For example, when both parents are fat, their children are predisposed to obesity with a possibility of 50% or higher. When either parent is obese, their children are reported to be predisposed to obesity with a possibility of about 25%.

Examples of other factors causing obesity include excessive caloric intake, insufficient exercise, low energy expenditure, etc.

The mainstay of treatment for obesity is lifestyle change, such as adopting an energy-limited diet, increasing exercise, increasing energy-consuming behavior, etc. Other therapies for obesity are drugs and surgery.

It is not easy for a person to change his or her lifestyle. Weight loss can be achieved by changing lifestyles, but to a limited degree. Typically, chemical therapy is adopted in combination with lifestyle change.

Drugs have long been used for the treatment of obesity.

Drugs for the treatment of obesity that target materials involved in the homeostatic control of energy in the body are clinically restricted because of their side effects.

There are two drugs that have received FDA permission for long-term use in the treatment of obesity; sibutramine, functioning as a serotonin-norepinephrine reuptake-inhibitor effective in the treatment of obesity, and orlistat, a lipase inhibitor is being used in the treatment of obesity.

Sibutramine, however, produces significant side effects, such as blood pressure increase, insomnia, xerostomia, vertigo, etc., and cannot be applied to patients suffering from cardiovascular diseases or uncontrolled hypertension. When orlistat is taken, the persons may suffer from diarrhea, fatty stool and constipation. In addition, orlistat is limitedly used because its efficacy is low in persons whose diets contain relatively small amounts of fat, such as for Asians.

Thus, active research has been conducted to develop naturally occurring physiologically active substances which can be applied to the treatment of obesity.

**[TABLE 1] Anti-obesity Agents and side effect**

| | |
|---|---|
| Anti-obesity drugs | Side effect |
| Thyroid hormone (1890) | Hyperthyroidism caused |
| Amphetamine (1930) | Addictive |
| Digitalis, diuretics (1960) | Death reported |
| Aminorex (1970) | Pulmonary hypertension caused |
| Fenfluramine (1990) | Valvular dysfunction caused in 35% of persons administered in combination with other therapy, sale prohibited in 1997. |
| Sibutramine (present) | Blood pressure increase, insomnia, xerostomia and vertigo. Prohibited to the patients with cardiovascular disease. |
| Orlistat (present) | Diarrhea, fatty stool, fecal incontinence. |

"Dong-Chung-Ha-Cho" (literally insect in winter but plant in summer) is a kind of medicinal fungus produced as a result of the parasitism of *Cordyceps millitaris* in insects. In high temperature and moisture conditions, the fungus infects living insects, proliferates therein to kill the host insects, and forms fruiting bodies on the surface of the host insects. Primarily, "Dong-Chung-Ha-Cho" refers to *Cordyceps sinensis,* a parasite on larvae of the Hepialidae family, but generally refers to all fungi attacking arthropods, such as spiders, at present.

Taxonomically, Dong-Chung-Ha-Cho belongs to the phylum *Ascomycota,* the order *Clavicipitales,* and the family *Clavicipitaceae* and is classified into three species: *Cordyceps, Podonectria* and *Torrubiella. Cordyceps* is the most popular Dong-Chung-Ha-Cho (Kobayasi Y., Trans Mycol. Soc. Japan, 23. 329-364, 1982). Approximately 800 kinds of *Dong-Chung-Ha-Cho* have been known to be distributed over the world thus far. Of them, 78 kinds have been collected and identified as with low host specificity and therefore utilizes various insect as a host. Generally, Dong-Chung-Ha-Cho is named after the insect hosts on which they grow. Naturally occurring Dong-Chung-Ha-Cho is too rare to acquire. In practice, various kinds of Dong-Chung-Ha-Cho are artificially cultured and are intensively studied with regard to the components and medical effects thereof.

*Cordyceps sinensis* is one of the most valuable herb medicines in the world. Wild-type *Cordyceps sinensis* is very rare and difficult to culture artificially. Chinese scientists developed artificially culturable Cs-4 strains as alternatives to *Cordyceps. Cordyceps sinensis* is known to have various medicinal effects including the ability to scavenge oxygen-free radicals, delay aging, stimulate endocrine, improve sexual functions, potentiate the respiratory system and the liver, and cancers and renal diseases (Zhu. J. S., J. Altern. Complement. Med., 4. 289-303 1998). *Cordyceps militaris* is another species of Cordyceps and widely used in Korea and China.

Cordycepin (3'-deoxyadenosine) is assumed to be the effective ingredient of *Cordyceps sinensis.* Long ago, Cunningham et al. isolated an inhibitor against the growth of *Bacillus subtilis* from *Cordyceps militaris.* This inhibitor was identified as cordycepin, (Kaczka, E. A., et al., Biochim. Biophys. Res. Commun., 14, pp. 456-457, 1964). Following the identification of cordycepin, various biological effects thereof are under study. For example, it has been proved that cordycepin plays an inhibitory role in bacteria, malaria, herpes, tumor, and leukemia.

Meanwhile, cordycepin has come to be known as a polyadenylation inhibitor, showing cytotoxicity against cancer cells. Recently, cordycepin was clinically tested as a therapeutic for leukemia (Eiichi N., et al., Biochemical Phar., Vol. 59: pp. 273-281, 2000).

However, cordycepin has never been reported or suggested to have an antiobesity effect.

Leading to the present invention, intensive and thorough research on treatment for obesity, conducted by the present inventors, resulted in the finding that cordycepin isolated from *Cordyceps militaris* inhibits the differentiation of 3T3-L1 cells into adipose cells, as well as the synthesis of triacylglycerol, This result indicates that cordycepin can be used as anti-obestic agent.

### Disclosure of the Invention

It is therefore an object of the present invention to provide a pharmaceutical composition for the treatment and prevention of obesity.

In order to achieve the above object, there is provided a pharmaceutical composition for use in the treatment and prevention of obesity, comprising cordycepin as an active ingredient.

### Brief Description of the Drawings

The above and other objects, features and other advantages of the present invention will be more clearly understood from the following detailed description taken in conjunction with the accompanying drawings, in which:
FIG. 1 is an HPLC profile of the active ingredient according to the present invention;
FIG. 2 is a ¹H NMR spectrum of the active material according to the present invention;
FIG. 3 is a ¹³C NMR spectrum of the active material according to the present invention;
FIG. 4 is a graph showing the dose-dependent inhibitory effect of cordycepin on the differentiation of 3T3-L1 cells into adipose cells (NC: negative control (DMEM); PC: positive control (MSD and MFD); C4: cordycepin 4 µM; C16: cordycepin 16 µM; C32: cordycepin 32 µM);
FIG. 5 is a graph showing the dose-dependent effect of cordycepin on the synthesis of triacylglycerol in 3T3-L1 cells (NC: negative control (DMEM); PC: positive control (MSD and MFD); C4: cordycepin 4 µM; C16: cordycepin 16 µM; C32: cordycepin 32 µM) ;
FIG. 6 shows the inhibitory activity of cordycepin and/or adenosine against the differentiation of 3T3-L1 cells into adipose cells (NC: negative control (DMEM); PC: positive control (MSD and MFD); C32: cordycepin 32 µH; A32: adenosine 32 µM; CA: cordycepin 32 µM + Adenosine 32 µM);
FIG. 7 is a photograph showing the effect of adenosine on cordycepin-induced inhibitory activity against triacylglycerol synthesis (NC: negative control (DMEM); PC: positive control (MSD and MFD); C32: cordycepin 32 µM; A32: adenosine 32 µM; CA: cordycepin 32 µM + Adenosine 32 µM) ; and
FIG. 8 is a photograph of Western blots showing the effects of cordycepin on the protein expression of C/EBPα, PPARγ, and anti-leptin in 3T3-L1 cells (NC: negative control (DMEM); PC: positive control (MSD and MFD); C32: cordycepin 32 µM; A32: adenosine 32 µM; CA: cordycepin 32 µM + Adenosine 32 µM).

### Best Mode for Carrying Out the Invention

Below, a detailed description is given in the present invention.

In accordance with the present invention, this provides a cordycepin-based pharmaceutical composition for the treatment and prevention of obesity.

Cordycepin, an active ingredient in the present invention, may be a commercially available form, or may be purified from *Cordyceps militaris.* However, it is encouraged to purify cordycepin from the mycelial cake or fruiting body of *Cordyceps militaris.*

In the present invention, cordycepin is found to have an effective inhibitory effect on the protein expression of C/EBPα, PPARγ, and anti-leptin, which are involved in adipogenesis and lipogenesis, thereby suppressing differentiation of fibroblast cells (3T3-L1 cells) into adipose cells. Also, cordycepin is proved to compete with adenosine, the precursor of ATP, NAD+, NADP+, CoA and FAD, therefore can serve as an effective inhibitor against the synthesis of triacylglycerol (TAG).

Also, cordycepin shows a dose-dependent inhibitory effect, both on the differentiation of fibroblast cells into adipocytes and on the biosynthesis of triacylglycerol.

Depending on the usage thereof, the composition of the present invention may comprise a pharmaceutically available vehicle or expedient, and may be formulated into dosage forms suitable for administration alone or in combination with other drugs. A thickening agent, a high in content of fiber additive, a capsulation agent, and a lipid may be useful as expedients in the present invention. Examples of these expedients are well known in the art.

The dose of the pharmaceutical composition of the present invention is dependent on the severity of disease, patient's state, age, sex, body condition, body weight, diet, excretion rate, administration time and route, etc.

In a preferable embodiment of the present invention, the active ingredient of the pharmaceutical composition is administered orally or non-orally in a single dose or in multiple doses within a daily total amount from 0.01 to 100 µg per kg of body weight. However, the dosage does not limit the present invention by no means.

A better understanding of the present invention may be obtained through the following examples, which are set forth to illustrate, but are not to be construed as the limit of the present invention.

### EXAMPLE 1: Isolation and Identification of Active Ingredient

*Cordyceps militaris* were purchased from Kyoungdong Herbal Medicinal Market, Seoul, Korea. After being dried, the *Cordyceps militaris* were grounded into powder and mixed with 10 ml/g of 80% ethanol. The suspension was extracted three times in a water bath with reflux system at 80°C for 3 hours, and dried in vacuuo to give a crude ethanol extract.

The crude ethanol extract was dissolved in distilled water and mixed with butanol at a ratio of 1:1 (v/v) and subjected to distribution chromatograph between the two different phases (3 times). The butanol extract was pooled and concentrated in vaccuo.

The residue thus obtained was purified by silica gel flash chromatography eluting with the solvent mixture of chloroform/methanol(7:3). The active fraction was condensed in a vacuum to completion, dissolved in a small amount of ethanol at a concentration of 20 µg/ml, and filtered through a Millipore filter (0.45 nm). Final purification was achieved by HPLC (column: ODS 20 x 250 mm; 15% methanol; diethylamine buffer pH 4.0; 10 ml/min) to afford an active fraction.

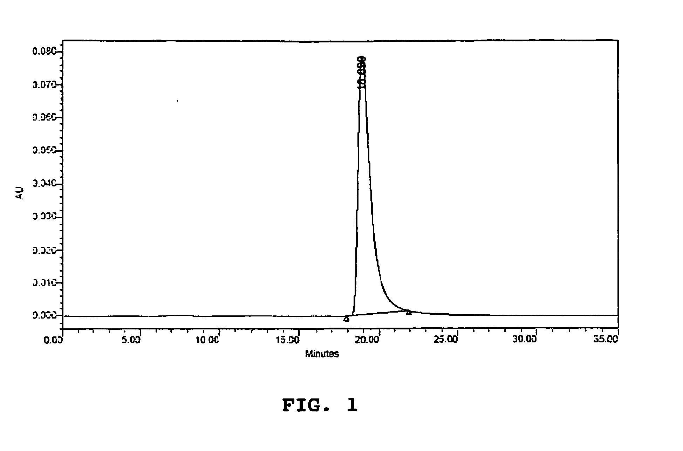

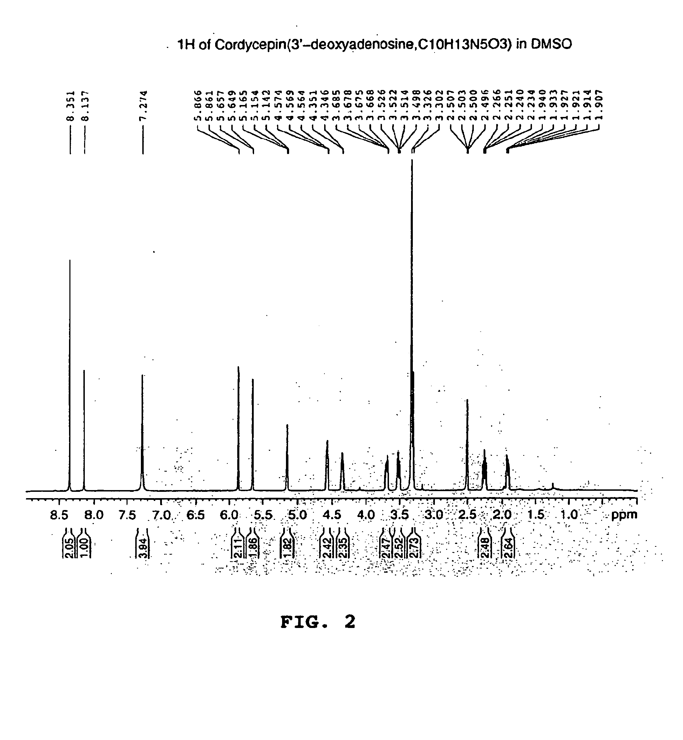

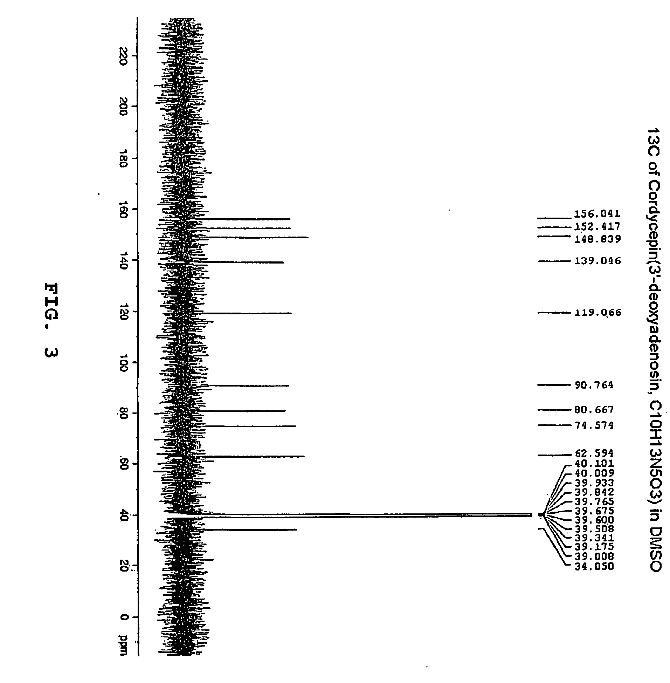

With reference to FIGS. 1 to 3, the HPLC profile, ¹H and ¹³C NMR spectra of the active substance according to the present invention are shown, respectively.

From the data, the active substance according to the present invention was identified to be 3'-deoxyadenosine (cordycepin), represented by the following chemical formula 1.

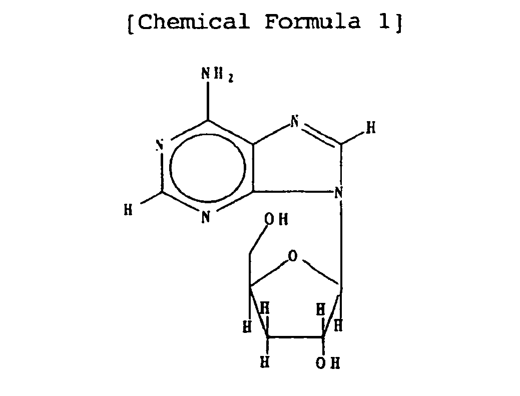

### EXPERIMENTAL EXAMPLE 1: Inhibitory Effect of Cordycepin on the Differentiation of Fibroblasts into Adipocytes

Fibroblast cells (3T3-L1 cells) were cultured in 10 cm-culture dishes containing animal cell culture media (refer to Table 2, below) at 37°C in a 5% CO₂ incubator, with the media replaced with fresh media every two or three days.

3T3-L1 cell line was purchased from the American Type Culture Collection (ATCC CL-173). The cells were grown and differentiated according to the protocol of Frost and Lane (Frost and Lane, 1985): 3T3-L1 preadipocytes were cultured in 100 mm plates containing a submerged medium for proliferation (DMEM supplemented with 10% BCS, 100 U/ml penicillin, and 100 µg/ml streptomycin) in an atmosphere of 5% CO₂ at 37°C. When the cell become confluent, the cells were harvested, splitted in a 6-well plate to a cell number of 4.0×10⁵ cells/ml and subsequently cultured until reaching confluence again. Two days after confluence, the cells were stimulated to differentiate into adipocytes by incubating for 2 days in the medium for stimulating differentiation (MSD, DMEM containing 1 µM DEX, 0.5 mM IBMX, and 10 µg/ml insulin), and maintained in the differentiated state in the medium for differentiation (MFD containing 10% BCS and 5 µg/ml insulin) for 7 days. MSD was freshly replaced every 48 hours and the comparison of the each medium composition used in this example was shown in Table 2.

Cordycepin was dissolved in the appropriate warm medium and added at the final concentration of 4, 16, and 32 µM.

**TABLE 2**

| Media | Glucose | BCS | FBS | Sodium bicarbonate | Insulin | Dex | IBMX |
|---|---|---|---|---|---|---|---|
| DMEM | 13.4 g/ℓ | 10% | - | 3.7g/ℓ | - | - | - |
| MSD | 13.4 g/ℓ | - | 10% | 3.7g/ℓ | 5 µg/mℓ | 1 µM | 0.5 mM |
| MFD | 13.4 g/ℓ | - | 10% | 3.7g/ℓ | 5 µg/mℓ | | |

| | | | | | | | |
|---|---|---|---|---|---|---|---|
| DMEM: Dulbecco's modified Eagle's medium BCS: Bovine calf serum FBS: Fetal Bovine serum DEX: Dexamethasone, IBMX: 1-methyl-3-isobutylxanthine. | | | | | | | |

Cells obtained in Example 1-1 were washed twice with PBS (pH 7.4), fixed with 10% formalin in PBS for 1 hour at room temperature and washed three times with distilled water. The cells were stained with filtered 0.5% oil red O for 10 min, and washed again with distilled water three times. The pictures of the stained cells were taken using an Olympus (CKX-41, Tokyo, Japan) microscope. The density of stained oil red O was measured using a spectrometer (DU-70, Beckman, Fullerton, CA) at 518 nm after the dye was extracted with propanol.

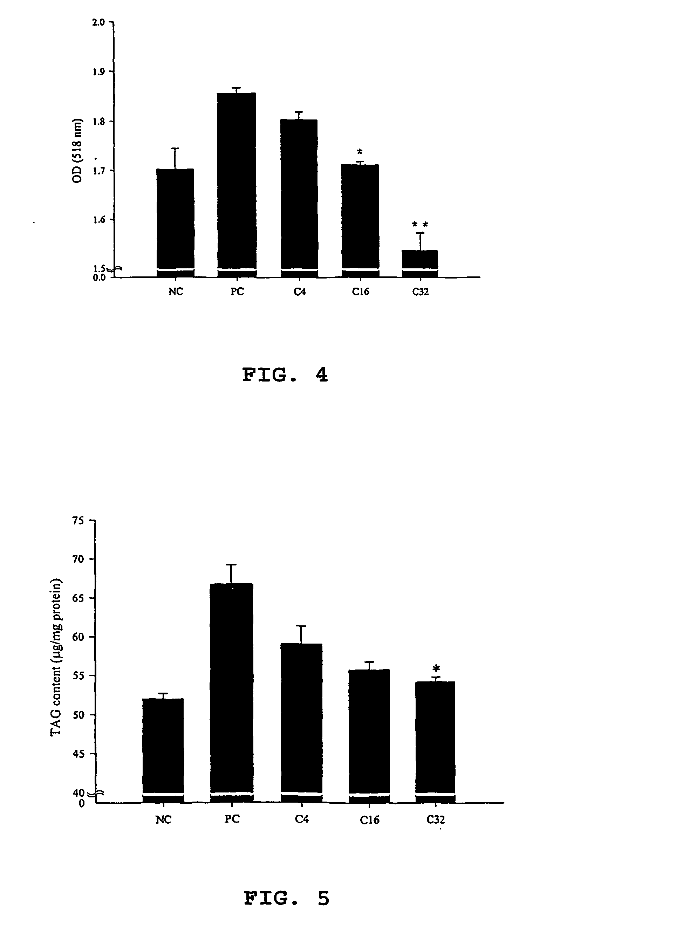

The results are given in FIG. 4. As shown in FIG. 4, cordycepin apparently inhibits the differentiation of 3T3-L1 cells into adipose cells in a dose-dependent manner. It was found that the differentiation of 3T3-L1 cells into adipocytes was almost completely suppressed in the presence of 32 µM of cordycepin.

### EXPERIMENTAL EXAMPLE 2: Inhibitory Effect on Triacylglycerol Synthesis

A measurement was made to find the effect of cordycepin on the synthesis of triacylglycerol (TAG). First, the cells in each group were detached using a Tris-EDTA buffer and disrupted with an ultrasonicator. A calibration curve for a standard solution was made according to the protocol of a BCA protein assay kit. The cell lysates were diluted in the kit reagent and warmed at 37°C for 30 min. Then, within 10 min, the solutions were measured for absorbance at 562 nm to quantify protein. Separately, according to the protocol of a BCS triacylglycerol kit, the cell lysates were diluted in the kit reagent and warmed at 37°C for 10 min. Then, within 60 min, the absorbance of the solutions was measured at 550 nm against a reagent blank. The amount of intracellular triacylglycerol per unit weight of protein (µg/mg) was calculated according to a calculation formula.

As can be clearly understood from FIG. 5, cordycepin inhibits the synthesis of triacylglycerol in 3T3-L1 cells in a dose-dependent manner.

### EXPERIMENTAL EXAMPLE 3: Effect of adenosine on the cordycepin-induced inhibition of differentiation and TAG synthesis

For the preliminary investigation of the mode of action of cordycepin on differentiation and TAG synthesis, adenosine, one of several cordycepin homologues, was added to the submerged medium for differentiation at the time of initiating differentiation at the same molar concentration as cordycepin (32 µM).

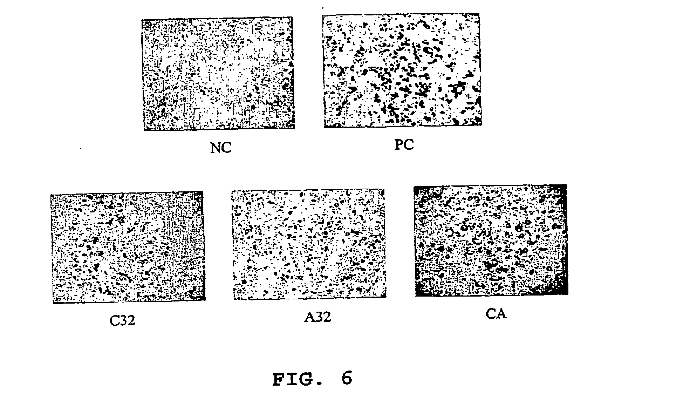

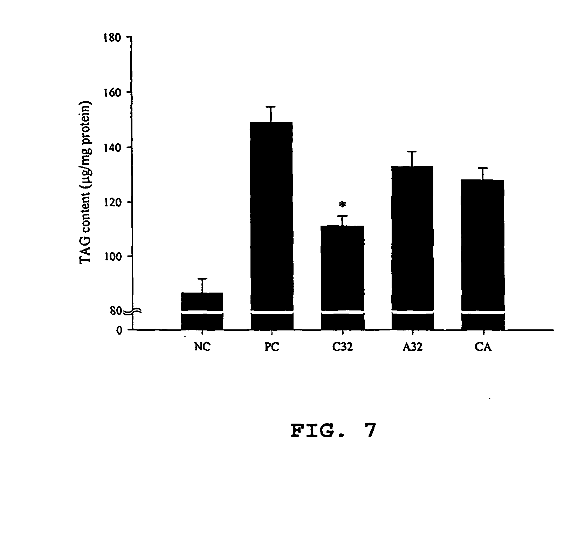

As shown in Fig. 6 and 7, the inhibitory effect of cordycepin on differentiation and TAG synthesis of 3T3-L1 cells was suppressed by adenosine.

This result suggests that cordycepin inhibits TAG synthesis by competing with adenosine, which is the precursor of ATP, NAD⁺, NADP⁺, CoA, and FAD.

### EXPERIMENTAL EXAMPLE 4: Western Blot Analysis

Western blot analysis was performed in order to examine the effect of cordycepin on the expression of C/EBPα, PPARγ, and anti-leptin, the essential factors involved in adipogenesis and lipogenesis.

3T3-L1 cells cultured in 6-well dishes were rinsed three times with ice-cold PBS, scraped into 0.5 ml of PBS, and centrifuged at 14,000 rpm for 1 min. Cell pellets were resuspended in 0.2 ml ice-cold RIPA buffer (50 mM Tris-HCl, pH 7.4, 150 mM NaCl, 1 mM EDTA, 1% NP 40, 0.25 % deoxycholate) and protease inhibitors (Complete Mini Protease Inhibitor Tablets, Roche, Indianapolis, IN). Suspensions were sheared through a 20-gauge needle, incubated on ice for 30 min and centrifuged at 14,000 rpm at 4°C for 20 min. Supernatants were collected and the protein concentration was determined using a BCA assay kit. Cell lysates were mixed with an equal volume of Laemmli SDS loading buffer (Bio-Rad Laboratories, Hercules, CA), equal amounts of lysate protein (30 mg) were loaded onto SDS-PAGE, and electrophoresis was carried out before the protein was transferred to a nitrocellulose membrane (Bio-Rad Laboratories, Hercules, CA). The membranes were blocked for 1 h in PBS containing 0.05% Tween-20 (USB corp., Cleveland, USA) and 5% nonfat dry milk. The membranes were incubated with antibodies against C/EBPα, PPARγ (Santa Cruz, CA) and anti-leptin (Sigma) for 1 hour, followed by three 10 min washes in PBS/Tween-20. The blots were subsequently treated with appropriate secondary antibodies conjugated to horseradish peroxidase for 1 hour at room temperature, and washed with PBS-T three times for 10 min. To visualize reaction complexes, an enhanced chemiluminescence system (ECL, Amersham International plc, Little Chalfont Buckinghamshire, England) was used, and reaction complexes were exposed to X-ray film.

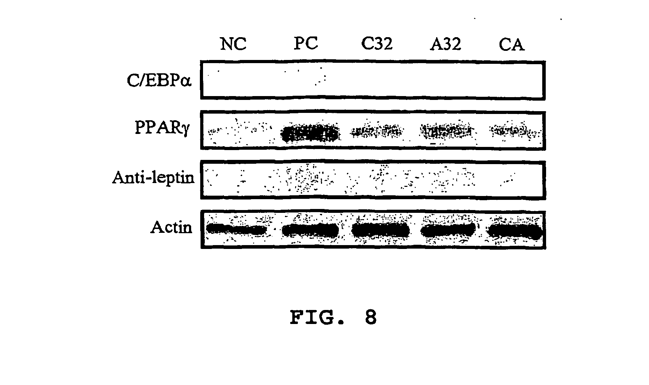

As shown in FIG. 8, the expression levels of C/EBPα and anti-leptin proteins in the NC cultured only in DMEM were almost negligible while the expression level of PPARγ in NC was significantly lower compared with that in PC. Cordycepin inhibited the expression levels of C/EBPα, PPARγ, and anti-leptin. In case of adenosine, it showed no significant effect on the expression levels of PPARα and anti-leptin in the differentiated 3T3-L1 cells, whereas it inhibited the C/EBPγ level.

Taken together, the data obtained in the above examples demonstrate that cordycepin suppresses adipocyte differentiation through PPARγ- and C/EBPα-mediated adipogenesis pathway, thus acting as an inhibitor against obesity.

### Industrial Applicability

As described above, cordycepin suppresses the expression of C/EBPα, PPARγ, and anti-leptin, the essential factors involved in adipogenesis and lipogenesis, thereby inhibiting the differentiation of fibroblast cells (3T3-L1 cells) into adipose cells and the synthesis of triacylglycerol. Therefore, cordycepin can be useful in the treatment and prevention of obesity as well as the relief and amelioration of symptoms.

Also, because Dong-Chung-Ha-Cho *(Cordyceps miliitaris)* is already permitted as food, the cordycepin extracted therefrom is free of toxicity and side effects. Consequently, the present invention can be used as an active ingredient of a pharmaceutical composition for the treatment and prevention of obesity and as an active ingredient of health food.

## Claims

1. A pharmaceutical composition for use in the treatment and prevention of obesity, comprising cordycepin as an active ingredient.

2. The pharmaceutical composition as set forth in claim 1, for use in said treatment, wherein the cordycepin is isolated and purified from *Cordyceps militaris.*

3. The pharmaceutical composition as set forth in claim 1, for use in said treatment, wherein the composition is administered orally or non-orally.

4. The pharmaceutical composition as set forth in claim 1, for use in said treatment, wherein the cordycepin inhibits the differentiation of fibroblast cells into adipocytes and the biosynthesis of triacylglycerol.

5. The pharmaceutical composition as set forth in one of claims 1 to 4, for use in said treatment, wherein the cordycepin suppresses adipocyte differentiation through PPARγ-and C/EBPα-mediated adipogenesis pathway, thus acting as an inhibitor against obesity.

## Patentansprüche

1. Pharmazeutische Zusammensetzung zur Verwendung bei der Behandlung oder Vorbeugung von Fettleibigkeit, umfassend Cordycepin als einen Wirkstoff.

2. Pharmazeutische Zusammensetzung nach Anspruch 1 zur Verwendung bei der genannten Behandlung, wobei das Cordycepin aus *Cordyceps militaris* isoliert und gereinigt ist.

3. Pharmazeutische Zusammensetzung nach Anspruch 1 zur Verwendung bei der genannten Behandlung, wobei die Zusammensetzung oral oder nicht oral verabreicht wird.

4. Pharmazeutische Zusammensetzung nach Anspruch 1 zur Verwendung bei der genannten Behandlung, wobei das Cordycepin die Differenzierung von Fibroblastzellen in Adipocyten und die Biosynthese von Triacylglycerol hindert.

5. Pharmazeutische Zusammensetzung nach einem der Ansprüche 1 bis 4 zur Verwendung bei der genannten Behandlung, wobei das Cordycepin Adipocytendifferenzierung durch PPARγ- und C/EBPα-vermittelten Adipogenesestoffwechselweg unterdrückt und **dadurch** als ein Inhibitor gegen Fettleibigkeit wirkt.

## Revendications

1. Composition pharmaceutique pour utilisation dans le traitement et la prévention de l'obésité, comprenant de la cordycépine comme principe actif.

2. Composition pharmaceutique selon la revendication 1, pour utilisation dans ledit traitement, où la cordycépine est isolée et purifiée à partir de *Cordyceps mi*/*itaris.*

3. Composition pharmaceutique selon la revendication 1, pour utilisation dans ledit traitement, où la composition est administrée par voie orale ou non orale.

4. Composition pharmaceutique selon la revendication 1, pour utilisation dans ledit traitement, où la cordycépine inhibe la différenciation des cellules fibroblastiques en adipocytes et la biosynthèse du triacylglycérol.

5. Composition pharmaceutique selon l'une quelque des revendications 1 à 4, pour utilisation dans ledit traitement, où la cordycépine supprime la différenciation adipocytaire par la voie de l'adipogénèse médiée par PPARγ et C/EBPα, agissant ainsi comme inhibiteur de l'obésité.
